# EUROPEAN PATENT SPECIFICATION

(11) **EP 2 009 015 B1**
(45) Date of publication and mention of the grant of the patent: **11.01.2012**
(21) Application number: 08010868.1
(22) Date of filing: 16.06.2008
(51) Int. Cl.: C07D 491/06

(54) **Process for the preparation of galantamine hydrobromide**
Herstellungsprozess für Galantaminhydrobromid
Procédé pour la préparation de bromhydrate de galantamine

(30) Priority: 18.06.2007 IN CH12572007
(43) Date of publication of application: 31.12.2008
(73) Proprietor: Aurobindo Pharma Limited, Hyderabad 500 038 (IN)
(72) Inventor: Koilpillai, Joseph Prabahar, Hyderabad 500 038, Andhra Pradesh (IN); Ganala, Naga Trinadhachari, Hyderabad 500 038, Andhra Pradesh (IN); Podila, Naveen Kumar, Hyderabad 500 038, Andhra Pradesh (IN); Upputuri, Venkata lakshmi, Hyderabad 500 038, Andhra Pradesh (IN); Dandala, Ramesh, Hyderabad 500 038, Andhra Pradesh (IN); Meenakshisunderam, Sivakumaran, Hyderabad 500 038, Andhra Pradesh (IN)
(74) Representative: Wittkopp, Alexander

(56) References cited:
- WO-A-97/11077
- US-A- 6 043 359

## Description

### FIELD OF THE INVENTION

The present invention relates to an improved process for the preparation of [4a*S*,6*R*,8a*S*]-4a,5,9,10,11,12-hexahydro-3-methoxy-11-methyl-6*H-*benzofuro[3a,3,2-*ef*][2]benzazepin-6-olof Formula I

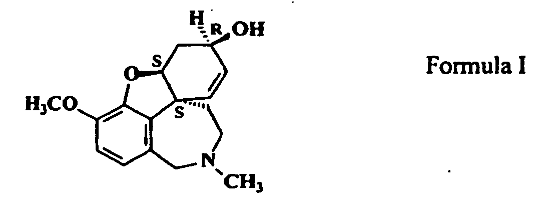

### BACKGROUND OF THE INVENTION

[4a*S*,6*R*,8a*S*]-4a,5,9,10,11,12-Hexahydro-3-methoxy-11-methyl-6*H-*benzofuro[3a,3,2-*ef*][2]benzazepin-6-ol (I) is generically known as Galantamine. Galantamine is a competitive and reversible acetyl cholinesterase inhibitor, which is approved for the treatment of mild to moderate Alzheimer's disease and is under development for other indications such as Vascular Dementia, Alzheimer's disease with cerebrovascular disease, mild cognitive impairment, schizophrenia, Parkinson's disease and other diseases wherein cognition is impaired. Galantamine (I) is commercially available as Razadyne^{®}.

Galantamine is a tertiary alkaloid, usually isolated from plants belonging to Amaryllidaceae family, for example, from galanthus species, such as the Daffodil, *Narcissus pseudomarcissus* and snowdrop or *Leucojum aestivum.* These plants are having Galantamine in concentrations of up to 0.3% with only small amounts of companion alkaloids, so that the extraction method can be used. This process of extraction is expensive and time consuming, hence not feasible for industrial scale operations.
Journal of Chemical Society (1962), 806-817 discloses the first synthetic process for the preparation of (-)-Galantamine from racemic Narwedine, which had been prepared in low yield from N-(3-hydroxy-4-methoxybenzyl)-N-methyl-2-(4-hydroxyphenyl)ethylamine or by oxidation of (±)-Galantamine to (±)-Narwedine followed by resolution and further reduction to (-)-Galantamine as shown below in Scheme I: 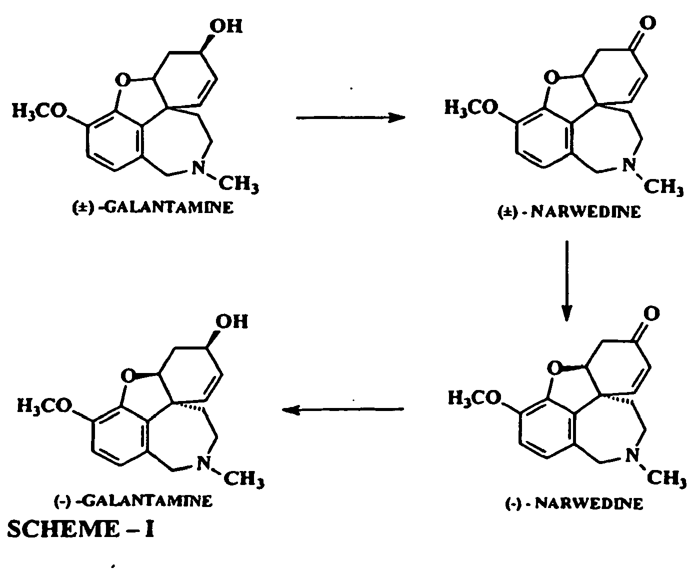 Journal of Heterocyclic Chemistry 25, 1809 (1988 ) discloses a process for the preparation of racemic Galantamine, which comprises reacting isovanillin with tyramine to produce N-(4-hydroxyphenethyl)-(3-hydroxy-4-methoxy)benzylamine (Norbelladine). N-Formylation followed by bromination of N-(4-hydroxyphenethyl)-(3-hydroxy-4-methoxy)benzylamine (Norbelladine) to obtain N-(4-hydroxyphenethyl)-N-(2-bromo-5-hydroxy-4-methoxy)benzylformamide (bromo formyl Norbelladine), which undergoes oxidative cyclization with potassium ferricyanide to produce a Narwedine type enone, which is further reduced and debrominated with lithium aluminium hydride to produce a mixture of racemic Galantamine and racemic Epigalantamine.

The process is as shown in Scheme-II:

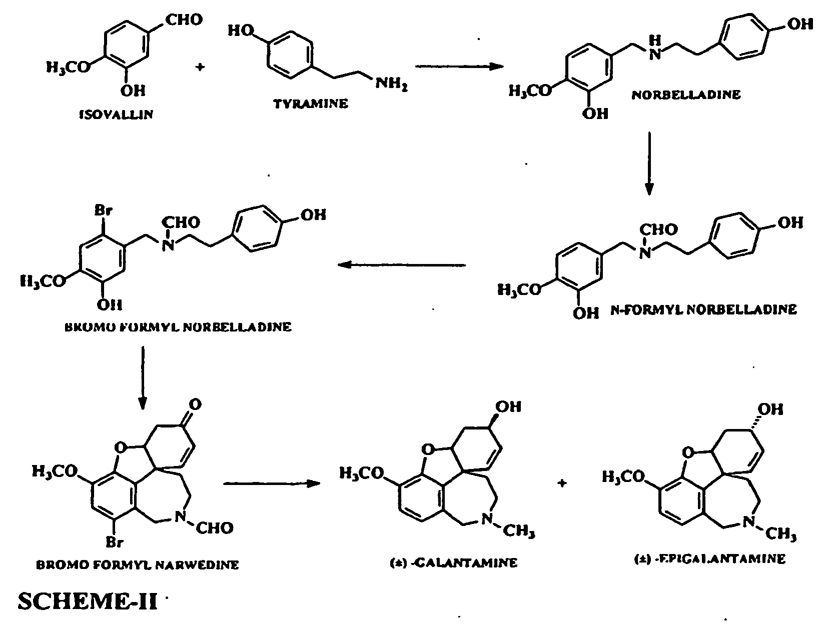

This process has several disadvantages, which limits its scope on industrial scale, due to the separation of Epigalantamine and Galantamine using column chromatography, which is tedious, laborious and involves usage of large quantities of solvents. Hence, it is industrially not suitable for the preparation of Galantamine.

Journal of Organic Chemistry 59, 5463, (1994), discloses a process for crystallizing racemic Narwedine in the presence of (+)-Galantamine from a solvent/amine base mixture, to give (-)-Narwedine. This publication discloses that seeding a solution of racemic Narwedine in an ethanol and triethylamine mixture with (-)-Narwedine results in the crystallization induced asymmetric transformation of (-)-Narwedine in 84% yield.

US 6,018,043 also discloses a process for the preparation of Galantamine by reducing Narwedine using LiAlH₄, NaBH₄ in presence of chiral modifiers to produce (-)-Galantamine.

The process is as shown in Scheme-III below:

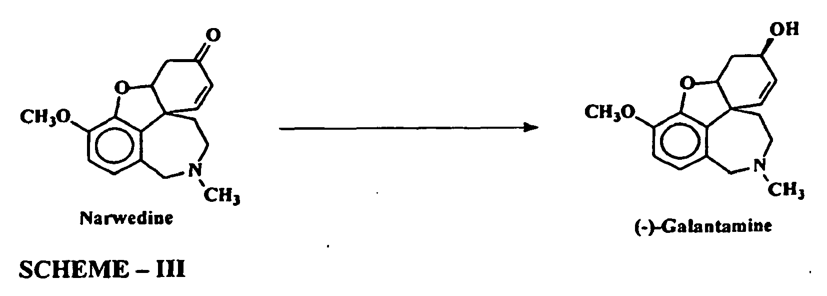

The above process affords Galantamine at an cxtcnt of 98% ee in 85% of yield, which is not suitable for commercial production of Galantamine.

Journal of Heterocyclic Chemistry (1995), 32, 195-199 discloses a process for the reduction of bromo formyl Nornarwedine using L-Selectride to produce bromo formyl Galantamine, which is further converted to Galantamine using LiAlH₄.

The process is as shown in Scheme-IV:

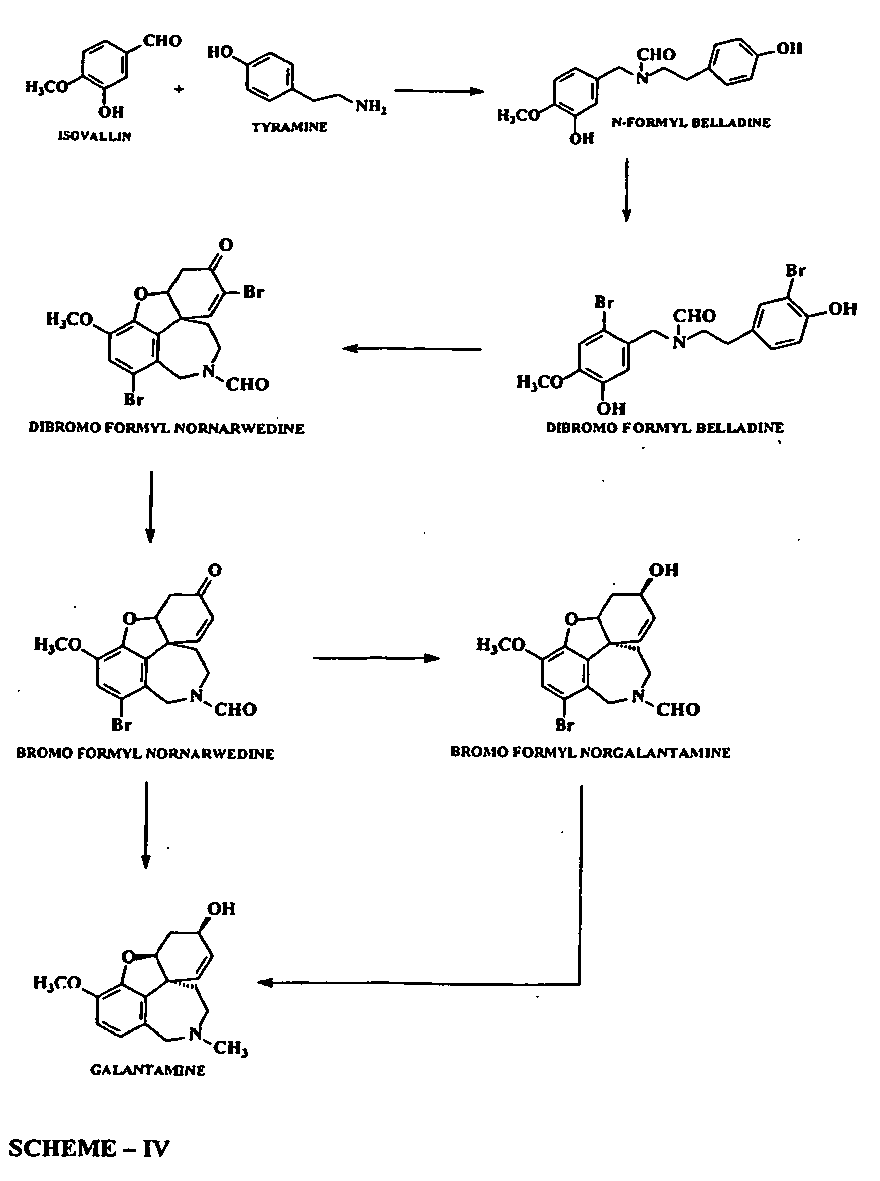

US 6,043,359 of Sanochemia, also discloses a process for the preparation of Galantamine by reducing bromo formyl Nornarwedine using L-Selectride to produce bromo Galantamine, which is debrominated using LiAlH₄ to produce Galantamine.

The process is as shown in Scheme-V:

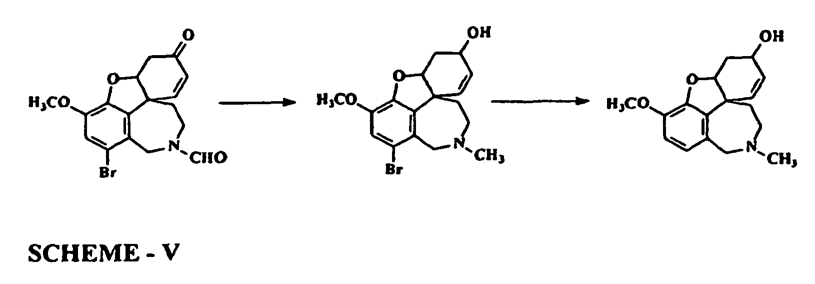

This process has the disadvantage that during stereoselective reduction, an undesired deformylation reaction occurs to yield bromo Norgalantamine, which requires an additional methylation step. Further, this reaction affords a racemic mixture of (±)-Galantamine, which has to be resolved and is not industrially desirable.

Janssen Pharmaceutica N.V patent US 6,346,618 B1, describes a process for the production of Galantamine by reducing the enantiomerically enriched diasteriomeric salt of Narwedine/bromo Narwedine to Galantamine/bromo Galantamine using NaBH₄/CeCl₃.

The process is as shown in Scheme-VI:

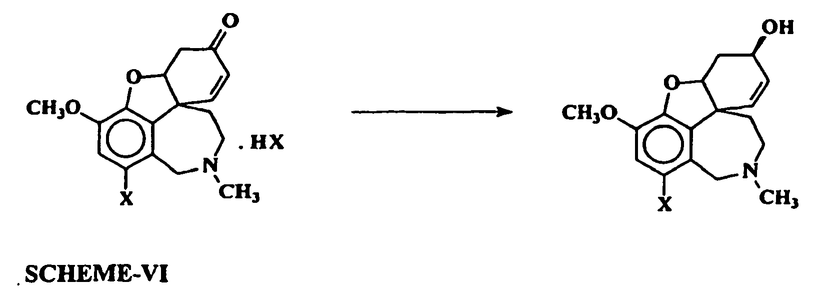

The reduction of Narwedine to Galantamine requires reagents that show 1,2-regioselectivity rather than 1,4-regioselectivity and that also give the required diastereoselectivity in the product, so that Galantamine is formed rather than Epigalantamine. There are several examples in the literature regarding the reagents that favor the 1,2-reduction over the 1,4-reduction of enones and which diastereoselectively reduce the carbonyl function in the desired manner. Such examples include LiAlH₄, aluminium triisopropoxide, NaBH₄ with catalytic amounts of rare earth metal halides.

The present invention is specifically directed towards the reduction of bromo formyl Nornarwedine using alkali metal borohydrides in the presence of rare earth metal halides to produce bromo formyl Galantamine isomers and bromo formyl Epigalantamine isomers, which in turn provides (-)-Galantamine in high yield and purity.

### OBJECTIVE OF THE INVENTION

The main objective of the present invention is to provide a simple and effective process for the preparation of Galantamine hydrobromide with high purity and improved yield on a commercial scale.

### SUMMARY OF THE INVENTION

Accordingly, the present invention relates to an improved process for the preparation of [4a*S*,6*R*,8a*S*]-4a,5,9,10,11,12-hexahydro-3-methoxy-11-methyl-6H-benzofuro[3a,3,2-ef][2] benzazepin-6-ol [Galantamine] of Formula I which comprises:
i) reducing 1-bromo-4a,5,9,10-tetrahydro-3-methoxy-6-oxo-6H-benzofuro [3a,3,2-ef][2]benzazepin-1(12H)carboxaldehyde (bromo formyl Nornarwedine) (II) 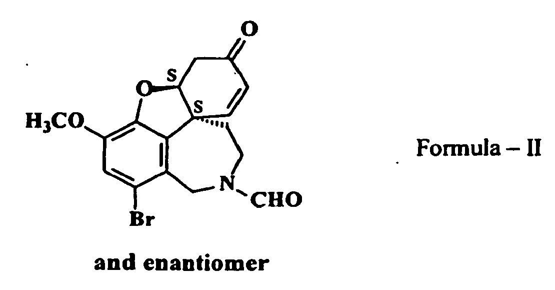 with alkali metal borohydride and rare earth metal halides in presence of a solvent to produce the mixture of 1-bromo-4a,5,9,10-tetrahydro-3-methoxy-6-hydroxy-*H*-benzofuro[3a,3,2-*ef*][2]benzazepin-11(12H)carboxaldehyde isomers ((±)-bromo formyl Epigalantamine) (IIIa) and 1-bromo-4a,5,9,10-tetrahydro-3-methoxy-6-hydroxy-6*H*-benzofuro[3a,3,2-*ef*][2]benzazepin-11 (12H)carboxaldehyde isomers ((±)-bromo formyl Galantamine) (IIIb) 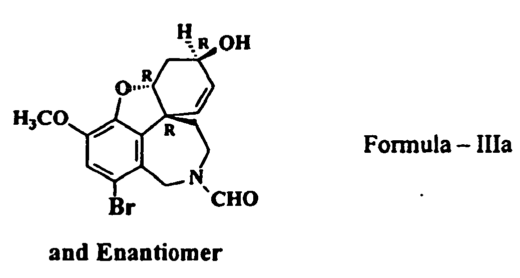 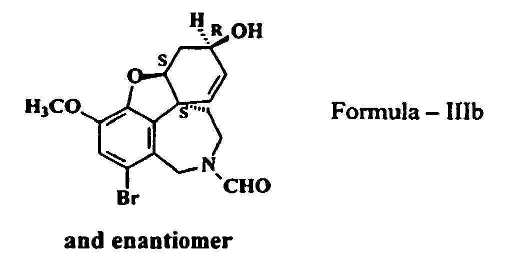
ii) reacting (±)-bromo formyl Epigalantamine (IIIa) and (±)-bromo formyl Galantamine (IIIb) with a reducing agent in presence of solvent to produce 4a,5,9,10,11,12-hexahydro-3-methoxy-11-methyl-6*H-*benzofuro[3a,3,2-*ef*][2] benzazepin-6-ol isomers ((±)-Epigalantamine) (IVa) and 4a,5,9,10,11,12-hexahydro-3-methoxy-11-methyt-6*H-*benzofuro[3a,3,2-*ef*][2]benzazepin-6-ol isomers ((±)-Galantamine mixture) (IVb) 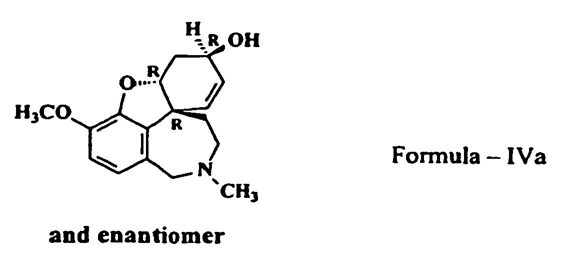 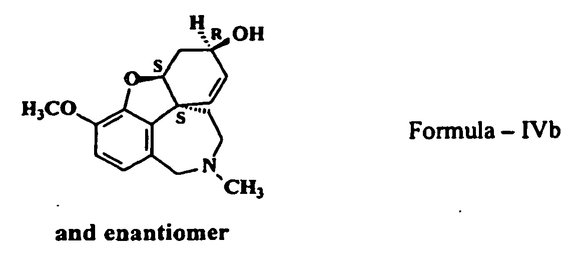
iii) oxidizing (±)-Epigalantamine (IVa) and (±)-Galantamine (IVb) with oxidizing agent in presence of solvent to produce 4a,5,9,10,11,12-hexahydro-3-methoxy-11-methyl-6*H*-benzofuro[3a,3,2-*ef*][2]benzazepin-6-one isomers [(±)-Narwedine] (V). 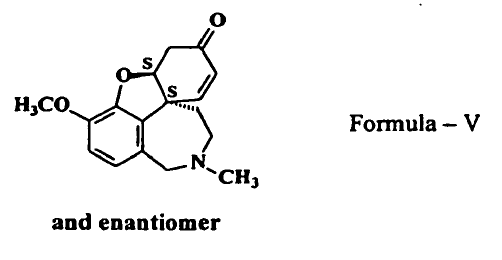
iv) treating (±)-Narwedine (V) with a base and (-)-Narwedine (VI) as a seed in presence of a solvent to produce [4a*S*,8a*S*]-4a,5,9,10,11,12-hexahydro-3-methoxy-11-methyl-6*H*-benzofuro[3a,3,2-*ef*][2]benzazepin-6-one [(-)-Narwedine] (VI) 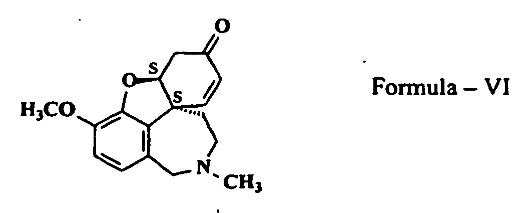
v) reducing (-)-Narwedine (VI) stereoselectively in presence of a solvent to produce [4a*S*,6*R*,8a*S*]-4a,5,9,10,11,12-hexahydro-3-methoxy-11-methyl-6*H*-benzofuro[3a,3,2-*ef*][2]benzazepin-6-ol [(-)-Galantamine] (I).

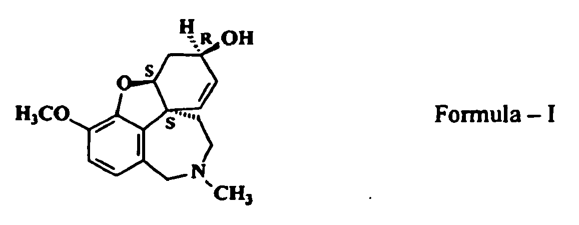

### DETAILED DESCRIPTION OF THE INVENTION

The present invention relates to an improved process for the preparation of [4a*S*,6*R*,8a*S*]-4a,5,9,10,11,12-hexahydro-3-methoxy-11-methyl-6*H-*benzofuro[3a,3,2-*ef*][2]benzazepin-6-ol [Galantamine] of Formula I.

1-Bromo-4a,5,9,10-tetrahydro-3-methoxy-6-oxo-6*H*-benzofuro[3a,3,2-*ef*][2]benzazepin 11(12H) carboxaldehyde (bromo formyl Nornarwedine) (II) is treated with alkali metal hydride/ alkaline earth metal hydride selected from NaBH₄, LiBH₄, AlH₃, LiAlH₄ and rare earth metal halides such as cerium, lanthanum, erbium salts in the presence of solvents selected from methylene chloride, ethylene chloride, methanol, ethanol, isopropanol or a mixture thereof. The reaction mass is cooled to a temperature of about 0-15°C and stirred for 30 min at room temperature. After completion of the reaction, water is added to the reaction mixture and filtered. The filtrate containing the mixture of (±)-bromo formyl Epigalantamine (IIIa) and (±)-bromo formyl Galantamine (IIIb) is concentrated and the product is extracted with a solvent selected from chloroform, toluene, methylene chloride, and ethyl acetate. The organic extract is concentrated to a residue, which contains the mixture of 1-bromo-4a,5,9,10-tetrahydro-3-methoxy-6-hydroxy-6*H*-benzofuro[3a,3,2-*ef*][2]benzazepin-11(12H) carboxaldehyde isomers ((±)-bromo formyl Epigalantamine) (IIIa) and 1-bromo-4a,5,9,10-tetrahydro-3-methoxy-6-hydroxy-6*H*-benzofuro [3a,3,2-*ef*][2]benzazepin-11(12H)carboxaldehyde isomers ((±)-bromo formyl Galantamine) (IIIb).

The mixture of 1-bromo-4a,5,9,10-tetrahydro-3-methoxy-6-hydroxy-6*H*-benzofuro[3a,3,2-*ef*][2]benzazepin-11-(12H)carboxaldehyde isomers ((±)-bromo formyl Epigalantamine) (IIIa) and 1-bromo-4a,5,9,10-tetrahydro-3-methoxy-6-hydroxy-6*H-*benzofuro[3a,3,2*-ef*][2]benzazepin-11-(12H)carboxaldehyde isomers ((±)-bromo formyl Galantamine) (IIIb) is reduced using a reducing agent selected from lithium aluminum hydride in presence of solvent selected from THF, ether, and diglyme, preferably THF at a temperature of about 25-35°C. The reaction mixture is refluxed at a temperature of about 55-60°C for about 1-2 h. The reaction mass is cooled to a temperature of about 0-35°C preferably to 5-10°C and the reaction can be continued optionally with purging air. After completion of the reaction, the reaction mixture is quenched and filtered. The filtrate is extracted with a solvent selected from dichloromethane, dichloroethane, chloroform, toluene, and xylene and the solvent is evaporated to produce a mixture of 4a,5,9,10,11,12-hexahydro-3-methoxy-11-methyl-6*H-*benzofuro[3a,3,2-*ef*][2]benzazepin-6-ol isomers ((±) Epigalantamine) (IVa) and 4a,5,9,10,11,12-hexahydro-3-methoxy-11-methyl-6*H*-benzofuro[3a,3,2-*ef*][2] benzazepin-6-ol isomers ((±) Galantamine) (IVb).

The mixture of racemic Epigalantamine and racemic Galantamine is oxidized with an oxidizing agent such as MnO₂ KMnO₄, oxalyl chloride/DMSO, or NaOCl/KBr/TEMPO preferably in MnO₂ in a solvent selected from chloroform, acetone, THF, toluene, at a temperature of about 25-30°C and is stirred for 10-30 h. After the completion of the reaction, the reaction mass is concentrated and the contents are stirred with chlorinated solvents such as dichloromethane, chloroform, CCl₄, toluene, xylene or ethylacetate. The contents are filtered and the filtrate is concentrated. The concentrated mass is triturated with an organic solvent selected from toluene, hexane, cyclohexane, and ethyl acetate, to crystallize 4a,5,9,10,11,12-hexahydro-3-methoxy-11-methyl-6H-benzofuro[3a,3,2-ef][2]benzazepin-6-one isomers ((±)-Narwedine) (V).

(±)-Narwedine (V) is treated with an organic base selected from trimethylamine, triethylamine and diisopropylethylamine, preferably triethylamine in presence of a solvent selected from methanol, ethanol, propanol, isopropanol, acetone and THF and at a temperature of about 60 - 90°C. The reaction mixture is cooled to a temperature of about 65-70°C and (-)-Narwedine seed is added to the solution. The resulting mixture is slowly cooled to a temperature of about 40-50°C and stirred continuously at the same temperature for about 3 days for complete dynamic resolution of (-)-Narwedine. The slurry of the reaction mass is cooled to a temperature of about 25-30°C and the product [4aS,8aS]-4a,5,9,10,11,12-hexahydro-3-methoxy-11-methyl-6H-benzofuro[3a,3,2-ef][2] benzazepin-6-one [(-)-Narwedine] (VI) is filtered and dried.

[4a*S*,8a*S*]-4a,5,9,10,11,12-Hexahydro-3-methoxy-11-methyl-6*H* benzofuro[3a,3,2-*ef*][2]benzazepin-6-one [(-)-Narwedine] (VI) is stereoselectively reduced using reducing agents selected from L-selectride, K-selectride, KS-selectride DIBAL-H, 9BBN-H, preferably L-selectride in presence of a solvent selected from THF, diglyme and ether at a temperature of about -20 to -15°C in 1hr. After the completion of the reaction, the reaction mixture is quenched. The reaction mixture is concentrated under reduced pressure and Galantamine is extracted using a solvent selected from dichloromethane, dichloroethane, chloroform, toluene, and ethyl acetate. The organic extract is concentrated under reduced pressure at a temperature of about 40-45°C to produce (-)-Galantamine base. Galantamine is treated with aqueous hydrobromic acid in a solvent selected from methanol, ethanol, aqueous methanol, aqueous ethanol, and aqueous THF a temperature of about 25-30°C for about 3h to obtain Galantamine hydrobromide. The product is filtered and dried.

The following examples illustrate the nature of the invention and are provided for illustrative purposes only and should not be construed to limit the scope of the invention.

### EXAMPLE 1:

### STEP I:

### Preparation of 1-bromo-4a,5,9,10-tetrahydro-3-methoxy-6-hydroxy-6H-benzofuro[3a,3,2-ef][2]benzazepin-11(12H)carboxaldehyde isomers and 1-bromo-4a,5,9,10-tetrahydro-3-methoxy-6-hydroxy-6H-benzofuro[3a,3,2-ef][2]benzazepin-11(12H)carboxaldehyde isomers [(±)-bromo formyl Epigalantamine and (±)-bromo formyl Galantamine mixture]

1-Bromo-4a,5,9,10-tetrahydro-3-methoxy-6-oxo-6H-benzofuro[3a,3,2-ef][2]benzazepin-11(12H) carboxaldehyde (bromo formyl Nornarwedine) (50g, 0.132 mol) and Cerium (III) chloride heptahydrate (49.28g, 0.132 mol) were dissolved in a mixture of methylene chloride/methanol (1:1, 1.5 L) and cooled to 0-5°C. Sodium borohydride (5g, 0.132 mol) was added over a period of 40 min to the reaction mixture at 0-5°C. The reaction mixture was stirred for 30 min at room temperature and the completion of the reaction was monitored by qualitative HPLC. After completion of the reaction, water (250 ml) was added to the reaction mixture and the resulting inorganic residue was filtered. The filtrate was concentrated under reduced pressure at 35-40°C and the product was extracted with methylene chloride (500 ml). The organic extract was concentrated to obtain the mixture of (±)-bromo formyl Epigalantamine and (±)-bromo formyl Galantamine mixture.

Chromatographic Purity >99.8% (by HPLC)

### STEP II:

Preparation of 4a,5,9,10,11,12-hexahydro-3-methoxy-11-methyl-6*H*-benzofuro[3a,3,2-*ef*][2]benzazepin-6-ol isomers and 4a,5,9,10,11,12-hexahydro-3-methoxy-11-methyl-6*H-*benzofuro[3a,3,2-*ef*][2]benzazepin-6-ol isomers [(±)-Epigalantamine and (±)-Galantamine mixture]

A solution of 1-bromo-4a,5,9,10-tetrahydro-3-methoxy-6-hydroxy-6*H*-benzofuro[3a,3,2-*ef*][2]benzazepin-11(12H)carboxaldehyde isomers and 1-bromo-4a,5,9,10-tetrahydro-3-methoxy-6-hydroxy-6*H*-benzofuro[3a,3,2-*ef*][2]benzazepin-11(12H)carboxaldehyde isomers ((±)-bromo formyl Epigalantamine and (±)-bromo formyl Galantamine mixture) (50g, 0.131 mol) in THF (500 ml) was added to a suspension of lithium aluminum hydride (17.48g, 0.46 mol) in THF (250 ml) at 25-35°C. The reaction mixture was refluxed at 55-60°C for 1h. Thereafter, the reaction mass was cooled to 25-30°C. Synthetic air (~80% N₂, ~20% O₂) was purged into the reaction mass with a flow rate of 600 ml/min while stirring for 2h and completion of the reaction was confirmed by qualitative HPLC. The reaction was quenched by adding DM water (18 ml), 15% (w/v) NaOH solution (18 ml) and DM water (54 ml). The reaction mass was stirred at 50-55°C for 30 min and the inorganic residue was removed by filtration. The filtrate was concentrated under reduced pressure and the product was extracted in chloroform (500 ml). The organic extract was concentrated at 35-40°C under reduced pressure to afford a mixture of (±)-Epigalantamine and (±)-Galantamine mixture.
Chromatographic Purity >96% (by HPLC)

### STEP III:

Preparation of 4a,5,9,10,11,12-hexahydro-3-methoxy-11-methyl-6*H*-benzofuro[3a,3,2-*ef*][2] benzazepin-6-one isomers [(±)-Narwedine] To a mixture of 4a,5,9,10,11,12-hexahydro-3-methoxy-11-methyl-6*H-*benzofuro[3a,3,2-*ef*][2] benzazepin-6-ol isomers and 4a,5,9,10,11,12-hexahydro-3-methoxy-11-methyl-6*H*-benzofuro [3a,3,2-*ef*][2]benzazepin-6-ol isomers [(±)-Epigalantamine and (±)-Galantamine mixture] (40g, 0.139 mol), active manganese dioxide (120g, 1.38 mol) was added in acetone (750 ml) at 25-30°C and stirred for 20h. The completion of the reaction was monitored by qualitative HPLC. After the completion of the reaction, the reaction mass was concentrated and contents were stirred with chloroform (500 ml). The contents were filtered to remove the inorganic residue and the filtrate was concentrated at 35-40°C under reduced pressure. The concentrated mass was triturated with ethyl acetate (100 ml) to crystallize the product. The product was filtered and dried under vacuum at 50-55°C.
Yield: 24g (60%)
Chromatographic Purity: >98.5%

### STEP IV:Preparation of [4aS,8aS]-4a,5,9,10,11,12-hexahydro-3-methoxy-11-methyl-6H-benzofuro [3a,3,2-ef][2]benzazepin-6-one [(-)-Narwedine]

4a,5,9,10,11,12-Hexahydro-3-methoxy-11-methyl-6H-benzofuro[3a,3,2-ef][2]benzaze-pin-6-one isomers [(±)-Narwedine] (30g, 0.105 mol) was dissolved in a mixture of ethanol (540 ml) and triethylamine (60 ml) at 75-80°C. The reaction mixture was cooled to 65-70°C and (-)-Narwedine (0.45g, 0.0016 mol) was added as a seed to the solution. The resulting mixture was slowly cooled to 40-42°C and stirred at 40-42°C for 3 days for complete dynamic resolution of (-)-Narwedine. The slurry reaction mass was cooled to 25-30°C. The obtained product was filtered and dried under vacuum at 50-55°C.
Yield: 27g (90%)
Chromatographic purity: > 99% (by HPLC)

### STEP V:

Preparation of [4a*S*,6*R*,8a*S*]-4a,5,9,10,11,12-hexahydro-3-methoxy-11-methyl-6H-benzofuro[3a,3,2-*ef*][2]benzazepin-6-ol [(-)-Galantamine hydrobromide]

Lithium tri-sec-butyl borohydride [L-selectride], (1 Molar solution in THF, 84.2 ml) was added drop wise to a uniform suspension of [4a*S*,8a*S*]-4a,5,9,10,11,12-hexahydro-3-methoxy-11-methyl-6H-benzofuro[3a,3,2-ef][2]benzazepin-6-one [(-)-Narwedine] (20g, 0.070 mol) in THF (100 ml) at -20 to -15°C for 30 min and the reaction mass was stirred at -20 to -15°C for 1h. The completion of the reaction was monitored by qualitative HPLC. The reaction mixture was quenched by adding aqueous hydrogen peroxide (40% w/w, 21.32g. 0.25 mol) and the excess peroxide was destroyed by stirring the reaction mass with aqueous sodium sulfite solution. The reaction mixture was concentrated under reduced pressure and extracted the product into chloroform (200 ml). The organic extract was concentrated under reduced pressure at 40-45°C to obtain Galantamine base. Galantamine base residue was dissolved in methanol (40 ml) and stirred with aqueous hydrobromic acid (48% w/w, 14.21g, 0.084 mol) at 25-30°C for 3 h to obtain Galantamine hydrobromide. The product was filtered and dried under vacuum at 50-55°C.
Yield: 22g (85.4%)
Chromatographic Purity: >99% (by HPLC)

### EXAMPLE 2:

Preparation of [4a*S*,8a*S*]-4a,5,9,10,11,12-Hexahydro-3-methoxy-11-methyl-6*H-*benzofuro [3a,3,2-ef][2]benzazepin-6-one [(-)-Narwedine]

4a,5,9,10,11,12-Hexahydro-3-methoxy-11-methyl-6*H*-benzofuro[3a,3,2-ef][2]benzazepin-6-one [(±)-Narwedine] (95 g, 0.333 mol) was dissolved in a mixture of DM water (72.2 ml), ethanol (1381 ml) and triethylamine (161.5 ml) at 75-80°C. The reaction mixture was cooled to 70-73°C and (-)-Narwedine (1.9 g, 0.0067 mol) was added as a seed to the solution. The resulting mixture was gradually cooled to 40-42°C and stirred at 40-42°C for 3 h for complete kinetic dynamic resolution of (-)-Narwedine. Then the slurry mass was cooled to 25-30°C and stirred for 1 h. The product was filtered and dried under vacuum at 55-65°C.
Yield: 85.5 g (90%)
Chromatographic purity: >99.5% (by HPLC)
Chiral purity: >99.5% (by HPLC)

### EXAMPLE 3:

Preparation of [4aS,6*R*,8a*S*]-4a,5,9,10,11,12-Hexahydro-3-methoxy-11-methyl-6*H* benzofuro[3a,3,2-ef][2]benzazepin-6-ol [(-)-Galantamine hydrobromide]

Lithium tri-sec-butyl borohydride [L-selectride], (1 molar solution in THF, 433.4 ml) was added drop wise to a uniform suspension of [4a*S*,8a*S*]-4a,5,9,10,11,12-hexahydro-3-methoxy-11-methyl-6*H*-benzofuro[3a,3,2-ef][2]benzazepin-6-one [(-)-Narwedine] (65 g, 0.228 mol) in THF (1300 ml) at -50 to -45°C for 1 h and stirred the reaction mass at -50 to -45°C for 1 h. The completion of the reaction was monitored by qualitative HPLC. The reaction mixture was quenched by adding hydrogen peroxide (40% w/w, 110.5 g, 1.3 mol) and the excess peroxide was destroyed by stirring the reaction mass with aqueous sodium sulfite solution. The reaction mixture was concentrated under reduced pressure and the product was extracted into toluene (1625 ml). The organic extract was concentrated under reduced pressure at 50-55°C to obtain Galantamine base. Galantamine base residue was dissolved in a mixture of ethanol (260 ml) and DM water (65 ml) and stirred with aqueous hydrobromic acid (48% w/w, 40.42 g, 0.24 mol) at 15-20°C for 2 h to obtain Galantamine hydrobromide. The product was filtered and dried under vacuum at 60-65°C.
Yield: 71.5 g (85.4%)
Chromatographic purity: >99.5% (by HPLC)
Chiral purity: >99.9% (by HPLC)

## Claims

1. A process for the preparation of [4aS,6R,8aS]-4a,5,9,10,11,12-hexahydro-3-methoxy-11-methyl-6H-benzofuro[3a,3,2-ef][2]benzazepin-6-ol [Galantamine] of Formula I which comprises:
i) reducing 1-bromo-4a,5,9,10-tetrahydro-3-methoxy-6-oxo-6H-benzofuro [3a,3,2-ef][2]benzazepin-11(12H)carboxaldehyde (bromo formyl Nomarwedine) (II) 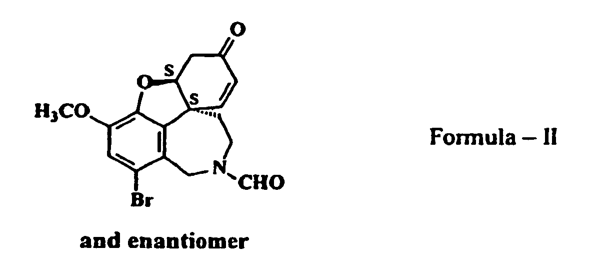 with an alkali metal borohydride and one or more rare earth metal halides in the presence of a solvent to produce the mixture of 1-bromo-4a,5,9,10-tetrahydro-3-methoxy-6-hydroxy-6*H*-benzofuro[3a,3,2-*ef*][2]benzazepin-11-(12H)carboxaldehyde isomers ((±)-bromo formyl Epigalantamine) (IIIa) and 1-bromo-4a,5,9,10-tetrahydro-3-methoxy-6-hydroxy-6*H*-benzofuro[3a,3,2-*ef*][2]benzazepin-11-(12H)carboxaldehyde isomers ((±)-bromo formyl Galantamine) (IIIb) 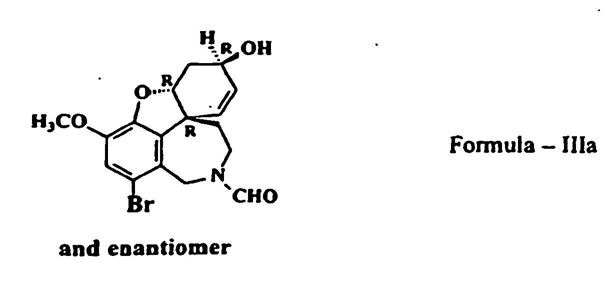 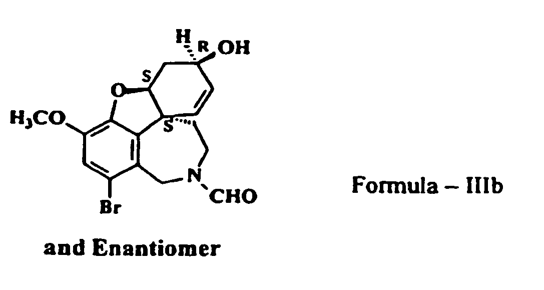
ii) reacting (±)-bromo formyl Epigalantamine (IIIa) and (±)-bromo formyl Galantamine (IIIb) with a reducing agent in the presence of a solvent to produce 4a,5,9,10,11,12-hexahydro-3-methoxy-11-methyl-6H-benzofuro[3a,3,2-ef][2]benzazepin-6-ol isomers ((±)-Epigalantamine) (IVa) and 4a,5,9,10,11,12-hexahydro-3-methoxy-11-methyl-6H-benzofuro[3a,3,2-ef][2]benzazepin-6-ol isomers and ((±)-Galantamine mixture) (IVb) 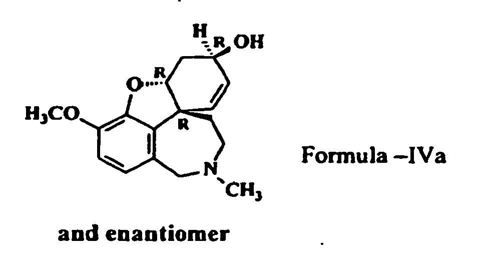 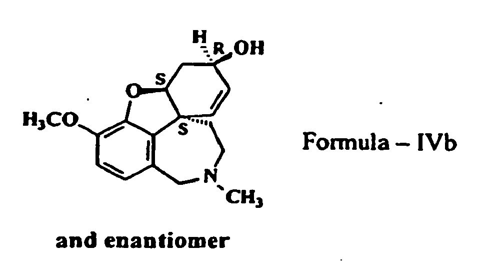
iii) oxidizing (±)-Epigalantamine (IVa) and (±)-Galantamine (IVb) with an oxidizing agent in presence of a solvent to produce 4a,5,9,10,11,12-hexahydro-3-methoxy-11-methyl-6H-benzofuro[3a,3,2-*ef*][2]benzazepin-6-one isomers [(±)-Narwedine] (V). 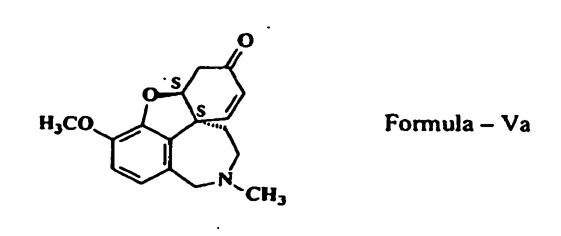 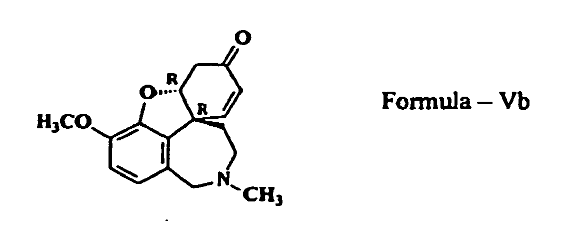
iv) treating (±)-Narwedine (Va) and (Vb) with a base and (-)-Narwedine (VI) as a seed in the presence of a solvent to produce [4aS,8aS]-4a,5,9,10,11,12-hexahydro-3-methoxy-11-methyl-6H-benzofuro[3a,3,2-ef][2]benzazepin-6-one [(-)-Narwedine] (VI) 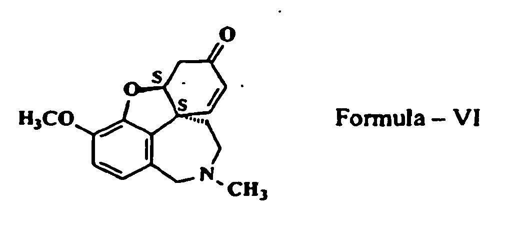
v) reducing (-)-Narwedine (VI) stereoselectively in the presence of a solvent to produce [4a*S*,6*R*,8a*S*]-4a,5,9,10,11,12-hexahydro-3-methoxy-11-methyl-6*H-*benzofuro[3a,3,2-*ef*]][2]benzazepin-6-ol [(-)-Galantamine] (I).

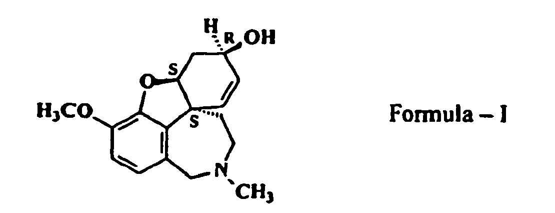

2. A process according to claim 1, wherein the alkali metal borohydride is selected from NaBH₄, and LiBH₄, and the rare earth metals halides are selected from cerium and lanthanum salts.

3. A process according to claim 1 or claim 2, wherein the solvent used in step (i) is selected from methylene chloride, dichloroethane, chloroform, methanol, ethanol, isopropanol, and a mixture thereof.

4. A process according to any one of claims 1-3, wherein the reducing agent used in step (ii) is selected from lithium aluminium hydride, sodium borohydride, and sodium cyanoborohydride.

5. A process according to any one of claims 1-4, wherein the solvent used in step (ii) is selected from THF, ether, and diglyme.

6. A process according to any one of claims 1-5, wherein the oxidizing agent used in step (iii) is selected from MnO₂, KMnO₄, oxalyl chloride/DMSO, and NaOCl/KBr/TEMPO; preferably MnO₂.

7. A process according to any one of claims 1-6, wherein the solvent used in step (iii) is selected from chloroform, acetone, THF, and toluene.

8. A process according to any one of claims 1-7, wherein the base used in step (iv) is selected from triethylamine, trimethylamine, and diisopropylcthylamine.

9. A process according to any one of claims 1-8, wherein solvent used in step (iv) is selected from methanol, ethanol, propanol, isopropanol, acetone, THF, water, and mixtures thereof.

10. A process according to any one of claims 1-9, wherein solvent used in step (v) is selected from chloroform, toluene, methylene chloride, ethyl acetate, and mixtures thereof.

## Patentansprüche

1. Verfahren zur Herstellung von [4aS,6R,8aS]-4a,5,9,10,11,12-Hexahydro-3-methoxy-11-methyl-6H-benzofuro[3a,3,2-ef][2]benzazepin-6-ol (Galantamin) der Formel I, das die Schritte umfasst:
i) Reduktion von 1-Brom-4a,5,9,10-tetrahydro-3-methoxy-6-oxo-6H-benzofuro-[3a,3,2-ef][2]benzazepin-11(12H)-carboxaldehyd (Bromformylnornarwedin) der Formel II 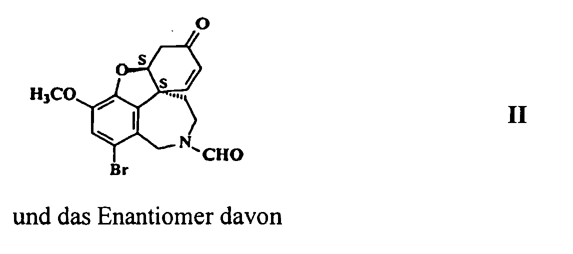 mit einem Alkalimetallborhydrid und einem oder mehreren Seltenerdmetallhalogeniden in Anwesenheit eines Lösungsmittels, um ein Gemisch der Isomere von 1-Brom-4a,5,9,10-tetrahydro-3-methoxy-6-hydroxy-6H-benzofuro[3a,3,2-ef][2]benzazepin-11(12H)-carboxaldehyd ((±)-Bromformylepigalantamin) der Formel IIIa und der Isomere von 1-Brom-4a,5,9,10-tetrahydro-3-methoxy-6-hydroxy-6H-benzofuro[3a,3,2-ef][2]benzazepin-11(12H)-carboxaldehyd ((±)-Bromformylgalantamin) der Formel IIIb 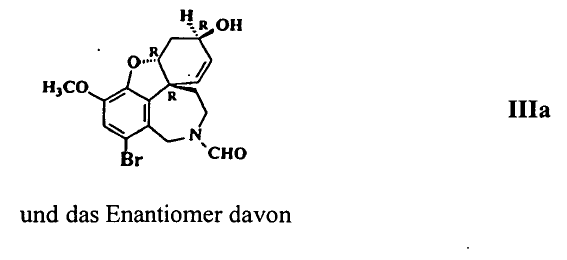 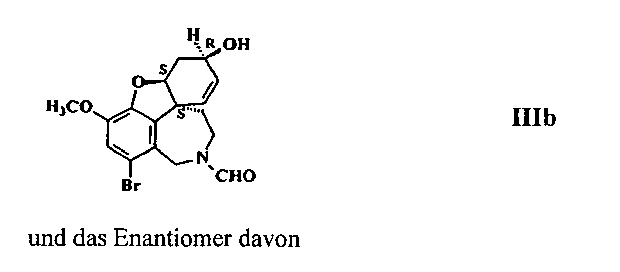 zu erzeugen,
ii) Reduktion von (±)-Bromformylepigalantamin (IIIa) und (±)-Bromformylgalantamin (IIIb) mit einem Reduktionsmittel in Anwesenheit eines Lösungsmittels, um die Isomere von 4a,5,9,10,11,12-Hexahydro-3-methoxy-11-methyl-6H-benzofuro[3a,3,2-*ef*][2]benzazepin-6-ol ((±)-Epigalantamin) der Formel IVa und die Isomere von 4a,5,9,10,11,12-Hexahydro-3-methoxy-11-methyl-6H-benzofuro[3a,3,2-*ef*][2]benzazepin-6-ol ((±)-Galantamingemisch) der Formel IVb 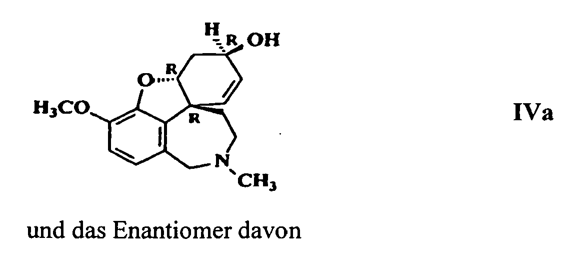 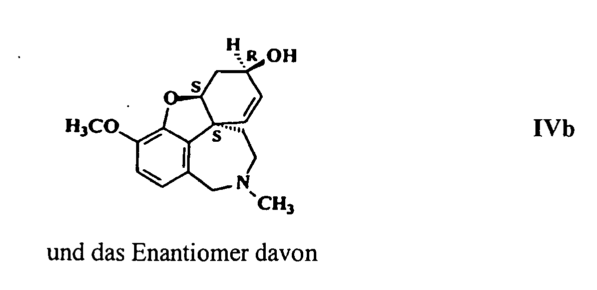 zu erzeugen,
iii) Oxidation von (±)-Epigalantamin (IVa) und (±)-Galantamin IVb mit einem Oxidationsmittel in Anwesenheit eines Lösungsmittels, um die Isomere von 4a,5,9,10,11,12-Hexahydro-3-methoxy-11-methyl-6H-benzofuro[3a,3,2-ef][2]benzazepin-6-on ((±)-Narwedin] der Formel V 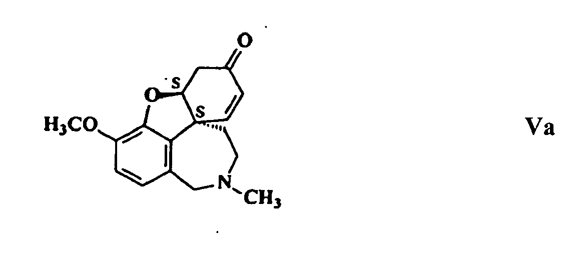 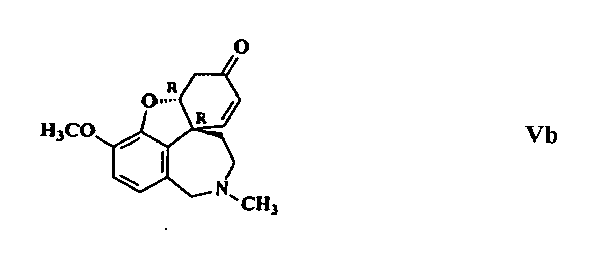 zu erzeugen,
iv) Behandeln von (±)-Narwedin (Va) und (Vb) mit einer Base und (-)-Narwedin (VI) als Impfkristall in Anwesenheit eines Lösungsmittels, um [4aS,8aS]-4a,5,9,10,11,12-Hexahydro-3-methoxy-11-methyl-6H-benzofuro[3a,3,2-ef][2]benzazepin-6-on ((-)-Narwedin) der Formel VI 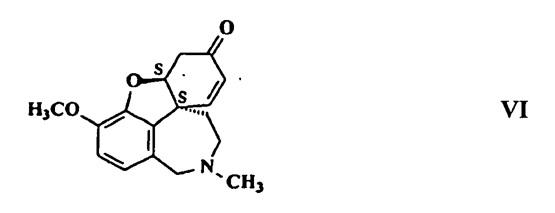 zu erzeugen,
v) stereoselektive Reduktion von (-)-Narwedin (VI) in Anwesenheit eines Lösungsmittels, um [4aS,6R,8aS]-4a,5,9,10,11,12-Hexahydro-3-methoxy-11-methyl-6H-benzofuro[3a,3,2-ef][2]benzazepin-6-ol ((-)-Galantamin) der Formel I 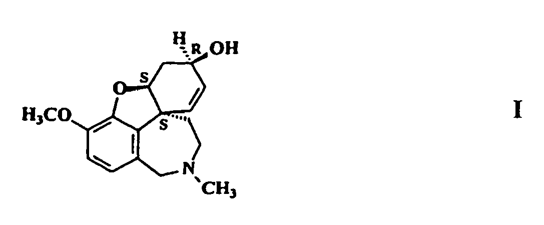 zu erzeugen.

2. Verfahren gemäß Anspruch 1, wobei das Alkalimetallborhydrid aus NaBH₄ und LiBH₄ ausgewählt ist, und das Seltenerdmetallhalogenid aus Cer- und Lanthansalzen ausgewählt ist.

3. Verfahren gemäß Anspruch 1 oder Anspruch 2, wobei das im Verfahrensschritt (i) verwendete Lösungsmittel aus Methylenchlorid, Dichlorethan, Chloroform, Methanol, Ethanol, Isopropanol und Gemischen davon ausgewählt ist.

4. Verfahren gemäß einem der Ansprüche 1-3, wobei das im Verfahrensschritt (ii) verwendete Reduktionsmittel aus Lithiumaluminiumhydrid, Natriumborhydrid und Natriumcyanoborhydrid ausgewählt ist.

5. Verfahren gemäß einem der Ansprüche 1-4, wobei das im Verfahrensschritt (ii) verwendete Lösungsmittel aus THF, Ether und Diglyme ausgewählt ist.

6. Verfahren gemäß einem der Ansprüche 1-5, wobei das im Verfahrensschritt (iii) verwendete Oxidationsmittel aus MnO₂, KMnO₄, Oxalylchlorid/DMSO und NaOCl/KBr/TEMPO, vorzugsweise MnO₂, ausgewählt ist.

7. Verfahren gemäß einem der Ansprüche 1-6, wobei das im Verfahrensschritt (iii) verwendete Lösungsmittel aus Chloroform, Aceton, THF und Toluol ausgewählt ist.

8. Verfahren gemäß einem der Ansprüche 1-7, wobei die im Verfahrensschritt (iv) verwendete Base aus Triethylamin, Trimethylamin und Diisopropylethylamin ausgewählt ist.

9. Verfahren gemäß einem der Ansprüche 1-8, wobei das im Verfahrensschritt (iv) verwendete Lösungsmittel aus Methanol, Ethanol, Propanol, Isopropanol, Aceton, THF, Wasser und Gemischen davon ausgewählt ist.

10. Verfahren gemäß einem der Ansprüche 1-9, wobei das im Verfahrensschritt (v) verwendete Lösungsmittel aus Chloroform, Toluol, Methylenchlorid, Ethylacetat und Gemischen davon ausgewählt ist.

## Revendications

1. Procédé de préparation de [4aS,6r,8aS]-4a,5,9,10,11,12-hexahydro-3-méthoxy-11-méthyl-6H-benzofuro[3a,3,2-ef][2]benzazépin-6-ol [galantamine] de formule I qui comprend les étapes suivantes consistant à :
i) réduire du 1-bromo-4a,5,9,10-tétrahydro-3-méthoxy-6-oxo-6H-benzofuro[3a,3,2-ef][2]benzazépine-11(12H)carboxaldéhyde (nomarwedine de bromoformyle) (II) 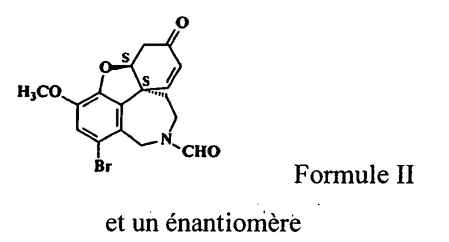 avec un borohydrure de métal alcalin et un ou plusieurs halogénures de métal du groupe des terres rares en présence d'un solvant pour produire le mélange d'isomères de 1-bromo-4a,5,9,10-tétrahydro-3-méthoxy-6-hydroxy-6*H-*benzofuro[3a,3,2-*ef*][2]benzazépine-11-(12H)carboxaldéhyde (épigalantamine de (±)-bromoformyle) (IIIa) et d'isomères de 1-bromo-4a,5,9,10-tétrahydro-3-méthoxy-6-hydroxy-6*H*-benzofuro[3a,3,2-*ef*][2]benzazépine-11-(12H)carboxaldéhyde (galantamine de (±)-bromoformyle) (IIIb) 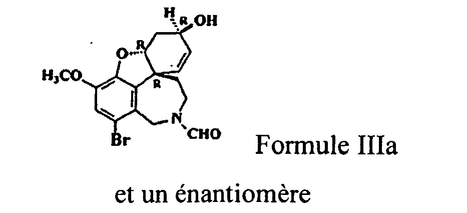 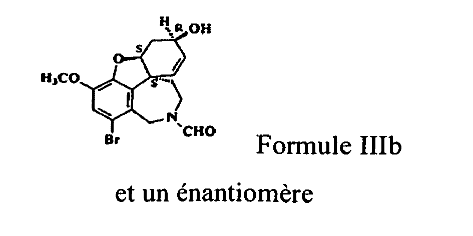
ii) faire réagir l'épigalantamine de (±)-bromoformyle (IIIa) et la galantamine de (±)-bromoformyle (IIIb) avec un agent réducteur en présence d'un solvant pour produire des isomères de 4a,5,9,10,11,12-hexahydro-3-méthoxy-11-méthyl-6H-benzofuro[3a,3,2-*ef*][2]benzazépin-6-ol ((±)-épigalantamine) (IVa) et des isomères de 4a,5,9,10,11,12-hexahydro-3-méthoxy-11-méthyl-6*H-*benzofuro[3a,3,2-*ef*][2]benzazépin-6-ol et (mélange de (±)-galantamine) (IVb). 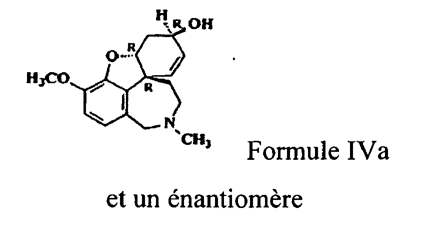 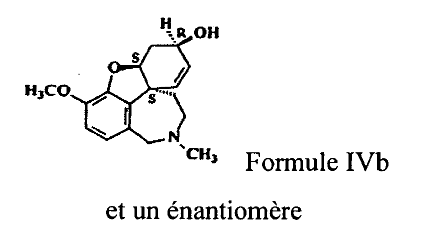
iii) oxyder (±)-épigalantamine (IVa) et (±)-galantamine (IVb) avec un agent oxydant en présence d'un solvant pour produire des isomères de 4a,5,9,10,11,12-hexahydro-3-méthoxy-11-méthyl-6*H*-benzofuro[3a,3,2-ef][2]benzazépin-6-one [(±)-narwedine] (V). 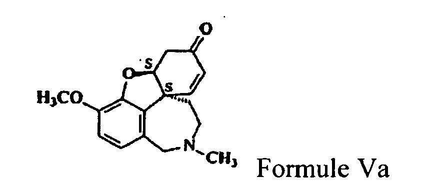 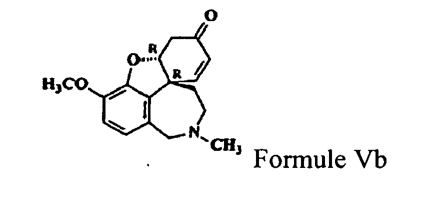
iv) traiter (±)-narwedine (Va) et (Vb) avec une base et de la (-)-narwedine (VI) comme amorce en présence d'un solvant pour produire du [4aS,8aS]-4a,5,9,10,11,12-hexahydro-3-méthoxy-11-méthyl-6H-benzofuro[3a,3,2-ef][2]benzazépin-6-one [(-)-narwedine] (VI) 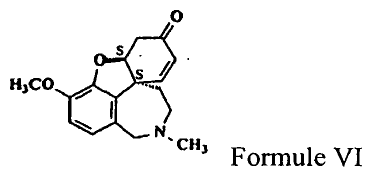
v) réduire la (-)-narwedine (VI) de manière stéréosélective en présence d'un solvant pour produire du [4a*S*,6*R*,8a*S*]-4a,5,9,10,11,12-hexahydro-3-méthoxy-11-méthyl-6*H*-benzofuro[3a,3,2-*ef*][2]benzazépin-6-ol [(-)-galantamine] (I)

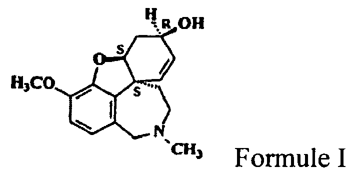

2. Procédé selon la revendication 1, dans lequel le borohydrure de métal alcalin est sélectionné parmi NaBH₄ et LiBH₄, et les halogénures de métaux du groupe des terres rares sont sélectionnés parmi des sels de cérium et de lanthane.

3. Procédé selon la revendication 1 ou 2, dans lequel le solvant utilisé dans l'étape (i) est sélectionné parmi chlorure de méthylène, dichloroéthane, chloroforme, méthanol, éthanol, isopropanol, et un mélange de ceux-ci.

4. Procédé selon l'une quelconque des revendications 1 à 3, dans lequel l'agent réducteur utilisé dans l'étape (ii) est sélectionné parmi hydrure de lithium-aluminium, borohydrure de sodium, et cyanoborohydrure de sodium.

5. Procédé selon l'une quelconque des revendications 1 à 4, dans lequel le solvant utilisé dans l'étape (ii) est sélectionné parmi THF, éther et diglyme.

6. Procédé selon l'une quelconque des revendications 1 à 5, dans lequel l'agent oxydant utilisé dans l'étape (iii) est sélectionné parmi MnO₂, KMnO₄, chlorure d'oxalyle/DMSO, et NaOCl/KBr/TEMPO ; de manière préférée MnO₂.

7. Procédé selon l'une quelconque des revendications 1 à 6, dans lequel le solvant utilisé dans l'étape (iii) est sélectionné parmi chloroforme, acétone, THF, et toluène.

8. Procédé selon l'une quelconque des revendications 1 à 7, dans lequel la base utilisée dans l'étape (iv) est sélectionnée parmi triéthylamine, triméthylamine, et diisopropyléthylamine.

9. Procédé selon l'une quelconque des revendications 1 à 8, dans lequel le solvant utilisé dans l'étape (iv) est sélectionné parmi méthanol, éthanol, propanol, isopropanol, acétone, THF, eau, et des mélanges de ceux-ci.

10. Procédé selon l'une quelconque des revendications 1 à 9, dans lequel le solvant utilisé dans l'étape (v) est sélectionné parmi chloroforme, toluène, chlorure de méthylène, acétate d'éthyle, et des mélanges de ceux-ci.
